(19) Europäisches Patentamt / European Patent Office / Office européen des brevets

(11) **EP 3 017 757 B1**

(12) **FASCICULE DE BREVET EUROPEEN**

(45) Date de publication et mention
de la délivrance du brevet:
**01.12.2021  Bulletin 2021/48**

(51) Int Cl.:
***A61B 5/024*** *(2006.01)*    ***A61B 5/00*** *(2006.01)*

(21) Numéro de dépôt: **15192010.5**

(22) Date de dépôt: **29.10.2015**

(54) **METHODE D'ELABORATION AUTOMATISEE D'UNE COURBE D'EVOLUTION D'UN INDICATEUR REPRESENTATIF DE LA VARIABILITE DE LA FREQUENCE CARDIAQUE D'UN ETRE HUMAIN OU D'UN ANIMAL DE COMPETITION**

METHODE ZUR AUTOMATISIERTEN ERZEUGUNG EINER EVOLUTIONSKURVE EINES REPRÄSENTATIVEN INDIKATORS DER HERZFREQUENZVARIABILITÄT EINES MENSCHEN ODER TIERES IN EINEM WETTKAMPF

AUTOMATED METHOD FOR PRODUCING A VARIATION CURVE OF AN INDICATOR REPRESENTING THE HEART-FREQUENCY VARIABILITY OF A HUMAN BEING OR A COMPETITION ANIMAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **07.11.2014  FR 1460786**

(43) Date de publication de la demande:
**11.05.2016  Bulletin 2016/19**

(73) Titulaire: **BE INVEST International S.A.
3372 Leudelange (LU)**

(72) Inventeur: **SABOUL, Damien
01130 LE POIZAT (FR)**

(74) Mandataire: **Marks & Clerk France
Immeuble "Visium"
22, avenue Aristide Briand
94117 Arcueil Cedex (FR)**

(56) Documents cités:
**EP-A1- 1 407 713**

- **KIM KO KEUN ET AL: "Effect of missing RR-interval data on nonlinear heart rate variability analysis", COMPUTER METHODS AND PROGRAMS IN BIOMEDICINE, vol. 106, no. 3, 11 novembre 2010 (2010-11-11), pages 210-218, XP028913729, ISSN: 0169-2607, DOI: 10.1016/J.CMPB.2010.11.011**
- **"Heart rate variability. Standards of measurement, physiological interpretation, and clinical use. Task Force of the European Society of Cardiology and the North American Society of Pacing and Electrophysiology", EUROPEAN HEART JOURNAL, OXFORD UNIVERSITY PRESS, GB, vol. 17, no. 3, 1 mars 1996 (1996-03-01), pages 354-381, XP002342325, ISSN: 0195-668X**

EP 3 017 757 B1

**Description**

La présente invention concerne le domaine de l'analyse de la variabilité de la fréquence cardiaque d'un individu ou d'un être humain ou d'un animal de compétition pour en déduire une information sur son état de forme ou de fatigue. Plus précisément, l'invention porte sur une méthode d'élaboration automatisée d'une courbe d'évolution d'un indicateur représentatif de la variabilité de la fréquence cardiaque d'un être humain ou d'un animal de compétition.

L'information déduite de l'analyse de la variabilité de la fréquence cardiaque reflète l'activité du système nerveux autonome, plus précisément l'influence des deux branches antagonistes de ce système que sont le système nerveux sympathique et le système nerveux parasympathique.

Cette information présente un intérêt dans le domaine de la médecine, notamment pour l'aide au diagnostic des maladies comprenant les troubles cardio-respiratoires, le diabète, le cancer, l'obésité, mais également dans le domaine de la détection du stress chez un individu ou encore dans le domaine du sport que ce soit le sport de haut niveau pour les êtres humains ou le domaine du sport animal ou des animaux de compétitions. Le sport animal comprend notamment les courses d'animaux, par exemple les courses de chevaux, de lévriers, de chameaux ou dromadaires mais aussi d'autres disciplines telles que le dressage, notamment le dressage de chevaux, le saut d'obstacle, le parcours. Par la suite, on utilise le terme animal de compétition pour désigner les exemples d'animaux précités ainsi que tout autre type d'animal de course non mentionné explicitement, ou de façon générale tout animal utilisé dans une compétition sportive ou artistique.

Dans le cas du sport, un indicateur de la variabilité de la fréquence cardiaque peut être utilisé comme indicateur de l'état de forme d'un sportif et pour améliorer et individualiser un entrainement d'un sportif, en particulier un sportif de haut niveau.

De la même façon, un tel indicateur peut être utilisé pour gérer l'entrainement d'un animal de compétition dans une discipline de sport animal.

Un problème à résoudre dans le domaine précité consiste à pouvoir fournir à un utilisateur, par exemple un sportif de haut niveau, sur requête, une visualisation de l'évolution de son état de forme, ou de l'état de forme d'un animal de compétition dans le cadre d'un sport animal, sur une période donnée. L'indicateur utilisé doit permettre un suivi de l'évolution dans le temps de l'état de forme de l'individu ou de l'animal de compétition.

L'art antérieur relatif au domaine de l'analyse de la variabilité de la fréquence cardiaque d'un être humain contient des méthodes de détermination d'indicateurs ponctuels de l'état de forme d'un individu.

Trois domaines d'analyse sont principalement connus : le domaine temporel, le domaine non-linéaire et le domaine fréquentiel.

Les méthodes connues d'analyse dans le domaine temporel sont basées sur des indicateurs statistiques déterminés directement à partir de mesures consécutives de l'intervalle de temps entre deux battements cardiaques. Les documents EP1407713A1, [1], [2] et [3] décrivent de telles méthodes d'analyse dans le domaine temporel.

Les méthodes d'analyse non-linéaire proposent des indicateurs qui sont fortement corrélés à ceux déterminés par les méthodes dans le domaine temporel. La référence [5] décrit une méthode d'analyse non-linéaire. L'intérêt principal de ces méthodes réside dans la représentation graphique des indices sous la forme de diagramme de Poincaré.

Les méthodes d'analyse dans le domaine fréquentiel sont les plus largement utilisées dans le domaine général de la santé car elles permettent de fournir un indicateur qui donne une information à la fois sur l'activité sympathique et parasympathique du système nerveux autonome de l'individu. Les méthodes dans le domaine fréquentiel sont basées sur des statistiques dérivées de la mesure de la variabilité de la fréquence cardiaque. Les documents [1] et [4] décrivent de telles méthodes. Une méthode fréquentielle consiste à mesurer la densité spectrale de puissance d'une courbe de variabilité de fréquence cardiaque et à séparer l'énergie en deux bandes de fréquences, hautes et basses. Les basses fréquences donnent une information sur l'influence sympathique du système nerveux autonome tandis que les hautes fréquences donnent une information sur l'influence parasympathique du système nerveux autonome. Le ratio entre les deux bandes de fréquences fourni un indicateur de l'activité sympatho-vagale.

L'art antérieur n'adresse cependant pas la problématique de l'automatisation de fourniture d'une courbe d'évolution de l'indice d'état de forme d'un individu pour pouvoir effectuer un suivi cohérent dans le temps de l'activité du système nerveux d'un individu. Les solutions connues ne permettent pas non plus une détection automatique, à partir d'indices d'état de forme, de périodes de forme ou de périodes de fatigue d'un individu. En effet, les indicateurs d'état de forme élaborés par les méthodes de l'art antérieur sont en général spécifiques à chaque individu. Il n'est donc pas possible d'élaborer une méthode automatisée qui permette le suivi de l'état de forme et la détection d'événements liés à la fatigue pour plusieurs individus, par exemple pour un groupe de sportifs.

L'invention fournit une solution au problème précité en proposant une méthode d'élaboration automatisée d'une courbe d'évolution d'un indicateur représentatif de la variabilité de la fréquence cardiaque d'un individu.

Une telle courbe peut notamment être utilisée par un entraineur dans le cadre du suivi d'entrainement d'un sportif de haut niveau. Elle permet, grâce au positionnement de seuils de détection automatique, de détecter des périodes de fatigue chez un individu. Un avantage de la méthode selon l'invention est qu'elle ne nécessite pas de configurer des seuils de détection propres à chaque individu. Grâce à une étape de normalisation, la méthode selon l'invention est adaptée à l'analyse de l'état de forme de plusieurs individus.

L'invention s'applique également à l'analyse de l'état de forme d'un animal de compétition tel qu'un cheval, un chien de course tel qu'un lévrier, un chameau ou un dromadaire.

L'invention a ainsi pour objet une méthode, mise en œuvre par ordinateur, d'élaboration automatisée d'une courbe d'évolution d'un indicateur représentatif de la variabilité de la fréquence cardiaque d'un individu, ou d'un animal de compétition, ladite méthode comprenant les étapes suivantes :

-   Détermination d'une séquence d'une pluralité de mesures d'un indicateur représentatif de la variabilité de la fréquence cardiaque, lesdites mesures étant réalisées avec une période temporelle constante prédéterminée, au moins une occurrence parmi lesdites mesures pouvant être omise, ladite séquence comprenant, pour chaque occurrence temporelle de ladite période, soit une mesure soit une valeur nulle correspondant à une absence de mesure à l'occurrence temporelle associée,
-   Normaliser les mesures de ladite séquence en excluant de la normalisation les valeurs nulles,
-   Effectuer au moins une interpolation de la séquence normalisée en effectuant une moyenne glissante sur une durée P prédéterminée des valeurs de la séquence, les valeurs nulles étant exclues de la moyenne,
-   Si au moins une valeur nulle, correspondant à une mesure absente, subsiste dans la séquence interpolée, filtrer ladite séquence en annulant les P-1 valeurs précédant ladite au moins une valeur nulle et les P-1 valeurs suivant ladite au moins une valeur nulle.

Selon un aspect particulier de la méthode selon l'invention, celle ci comprend deux interpolations successives de la séquence normalisée.

Selon un aspect particulier de la méthode selon l'invention, ledit indicateur est un indice représentatif de l'activité sympatho-vagale d'un individu obtenu en exécutant les étapes suivantes :

-   Recevoir une séquence comportant une pluralité de mesures, dites mesures R-R, de l'intervalle de temps entre deux battements cardiaques consécutifs de l'individu,
-   Construire une première sous-séquence constituée des écarts positifs entre deux valeurs consécutives de la dite séquence reçue,
-   Construire une seconde sous-séquence constituée des valeurs absolues des écarts négatifs entre deux valeurs consécutives de la dite séquence reçue,

-   Calculer un indice égal à une valeur représentative de la comparaison entre le résultat d'une fonction statistique appliquée aux valeurs de la première sous-séquence et le résultat de ladite fonction statistique appliquée aux valeurs de la seconde sous-séquence.

Selon un aspect particulier de la méthode selon l'invention, l'indice calculé est égal au ratio entre une fonction statistique appliquée aux valeurs de la première sous-séquence et ladite fonction statistique appliquée aux valeurs de la seconde sous-séquence.

Selon un aspect particulier de la méthode selon l'invention, ladite fonction statistique est prise dans l'ensemble suivant : une moyenne, une variance, un écart type.

Selon un aspect particulier de la méthode selon l'invention, ledit indicateur est égal à une mesure de la fréquence cardiaque moyenne de l'individu ou une mesure statistique déterminée à partir d'une pluralité de mesures de l'intervalle de temps entre deux battements cardiaques consécutifs de l'individu.

Selon un aspect particulier de la méthode selon l'invention, ledit indicateur est égal à une mesure, dans au moins une sous-bande fréquentielle, de la densité spectrale de puissance d'une pluralité de mesures de l'intervalle de temps entre deux battements cardiaques consécutifs de l'individu.

Selon un aspect particulier de la méthode selon l'invention, celle-ci comprend en outre

-   La transmission d'une requête d'établissement de ladite courbe sur une période de temps donnée par un individu,
-   L'affichage de ladite courbe sur un écran.

L'invention a également pour objet une utilisation d'une courbe d'évolution d'un indicateur représentatif de la variabilité de la fréquence cardiaque d'un individu obtenue par exécution de la méthode selon l'invention comprenant la configuration d'au moins un seuil de détection d'un état de l'individu parmi un état de forme ou un état de fatigue et l'application dudit au moins un seuil à ladite courbe pour détecter ledit état. Selon un mode de réalisation particulier, l'invention s'applique également en remplaçant l'individu par un animal de compétition, par exemple un cheval, un chien de course, tel qu'un lévrier, ou un chameau ou un dromadaire.

L'invention a également pour objet un programme d'ordinateur comportant des instructions pour l'exécution de la méthode d'élaboration automatisée d'une courbe d'évolution d'un indicateur représentatif de la variabilité de la fréquence cardiaque d'un individu selon l'invention, lorsque le programme est exécuté par un processeur et un support d'enregistrement lisible par un processeur sur lequel est enregistré un programme comportant des instructions pour l'exécution de la méthode d'élaboration automatisée d'une courbe d'évolution d'un indicateur représentatif de la variabilité de la fréquence cardiaque d'un individu selon l'invention, lorsque le programme est exécuté par un processeur.

L'invention a encore pour objet un dispositif, par exemple terminal ou serveur, comprenant une mémoire et un processeur configurés pour mettre en œuvre la méthode d'élaboration automatisée d'une courbe d'évolution d'un indicateur représentatif de la variabilité de la fréquence cardiaque d'un individu selon l'invention.

Selon une variante particulière, le dispositif selon l'invention comprend en outre :

- Des moyens pour recevoir une requête d'établissement de ladite courbe sur une période de temps donnée par un individu,
- Des moyens pour transmettre ladite courbe vers un écran d'affichage.

D'autres caractéristiques et avantages de la présente invention apparaîtront mieux à la lecture de la description qui suit en relation aux dessins annexés qui représentent :

- La figure 1a, un organigramme détaillant les étapes de mise en œuvre de la méthode d'élaboration d'une courbe d'évolution d'un indicateur représentatif de la variabilité de la fréquence cardiaque d'un individu,
- La figure 1b, un schéma illustrant, sur un exemple, l'allure des séquences d'indice obtenues à chaque étape de la méthode de la figure 1a,
- La figure 2, un diagramme illustrant un exemple de courbe d'évolution de l'état de forme d'un individu obtenu à l'issue de la méthode décrite à l'appui de la figure 1a,
- La figure 3, un organigramme détaillant les étapes de mise en œuvre d'une méthode de détermination d'un indice représentatif de l'activité sympatho-vagale d'un individu,
- Les figures 4a et 4b deux diagrammes illustrant l'influence des étapes de filtrage appliquées à une séquence de mesures R-R afin de supprimer les artefacts de mesures,
- La figure 5, un schéma illustrant une mise en œuvre possible de l'invention.

Un mode de réalisation de l'invention est tout d'abord décrit en détail pour son application à la surveillance de l'état de forme d'un être humain.

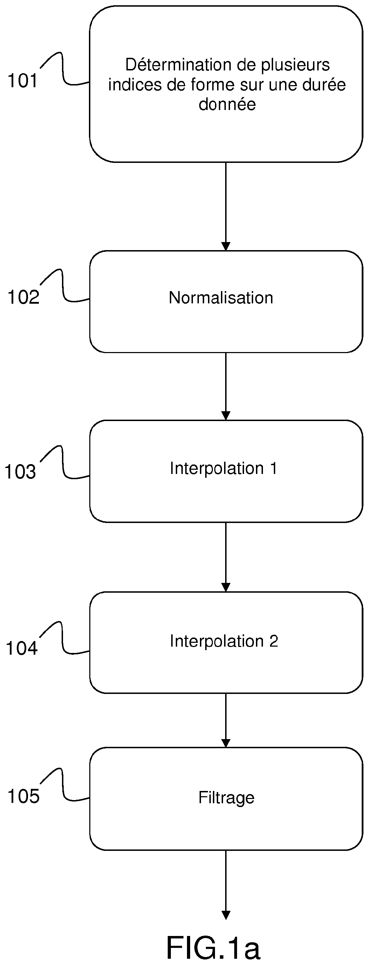

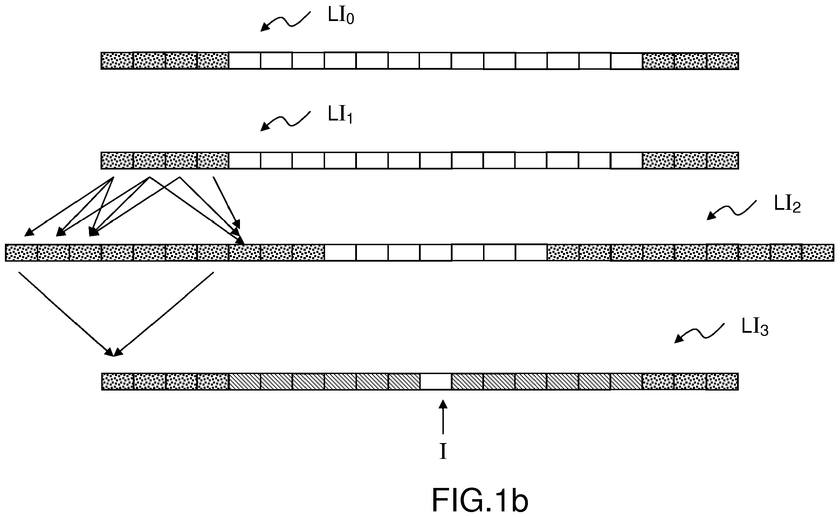

Les figures 1a et 1b illustrent les étapes de mise en œuvre de la méthode d'élaboration d'une courbe d'évolution d'un indicateur représentatif de la variabilité de la fréquence cardiaque d'un individu. On décrit dans un premier temps la méthode d'élaboration de la courbe d'évolution sans développer la nature précise de l'indicateur ponctuel utilisé. Dans un second temps on explicite plusieurs indicateurs particuliers.

Selon une première étape 101 de la méthode selon l'invention, on calcule une pluralité d'indicateurs ou indices sur une durée donnée pour le même individu. Les indices sont calculés à une période fixe, par exemple une période égale à un jour ou plusieurs jours. Cependant, à certaines occurrences de la période, les calculs d'indices peuvent être omis. Un indicateur est calculé à partir d'une pluralité de mesures de l'intervalle entre deux battements cardiaques consécutifs d'un individu, encore appelées mesures R-R. Le nombre d'indices de forme calculés dépend notamment du nombre d'acquisitions de mesures R-R effectuées par l'individu. Par exemple, l'individu peut effectuer une séance d'acquisition par jour mais peut omettre cette séance certains jours.

A l'issue de la première étape 101, on obtient une première liste d'indices $LI_0$. Sur la figure 1b, la liste $LI_0$ est représentée par une succession de cases. Les cases grisées correspondent aux périodes où un indice a effectivement été calculé tandis que les cases blanches correspondent à des périodes où aucun indice n'a été calculé. Dans l'exemple de la figure 1b, on considère une période d'acquisition égale à un jour et une séquence de durée totale égale à 20 jours. Selon cet exemple, une période continue de 13 jours intervient pendant laquelle aucune acquisition de mesures R-R et aucun calcul d'indice n'a été effectué. Cet exemple est donné à titre purement illustratif et nullement limitatif. Un autre scénario consisterait à effectuer un calcul d'indice tous les jours pendant les 20 jours.

Cette première liste est ensuite normalisée 102. La normalisation consiste à calculer la moyenne M des valeurs non nulles de la première liste $LI_0$ et son écart type σ puis à effectuer l'opération de normalisation suivante :

$$I_{norm}= (I\text{-}M)/ σ$$

Pour effectuer l'opération de normalisation, les cases de la première liste $LI_0$ qui présentent des valeurs nulles ne sont pas prises en compte.

On obtient une deuxième liste normalisée $LI_1$ qui comporte autant d'éléments que la première liste $LI_0$. L'opération de normalisation permet d'obtenir des valeurs d'indice de forme qui peuvent être comparées sur différentes périodes de temps et qui prennent en compte les critères physiques de l'individu. Cette étape de normalisation est une étape essentielle de l'invention qui permet d'élaborer une courbe d'évolution d'un indicateur de l'état de forme ou de fatigue d'un individu. Notamment grâce à cette étape, il est possible d'élaborer une courbe pour différents individus ayant des caractéristiques physiques différentes. Comme cela sera explicité par la suite, l'invention permet de fixer des seuils de détection d'état de fatigue qui sont communs à plusieurs individus.

Dans une troisième étape 103, on effectue une première interpolation des éléments de la liste normalisée $LI_1$. L'interpolation présente un paramètre P égal au nombre de valeurs sur laquelle l'interpolation est effectuée. Le paramètre P est configuré en prenant en compte les caractéristiques du profil de l'individu dont on souhaite évaluer la courbe de forme. Par expérimentation, le paramètre P est pris égal à 7 jours pour le cas d'un sportif de haut niveau. Le paramètre P est préférentiellement un nombre entier impair.

Pour construire la liste interpolée $LI_2$, on ajoute tout d'abord (P-1)/2 éléments en début et en fin de liste. Chaque élément de la liste interpolée est calculé comme la moyenne glissante de P éléments de la liste normalisée $LI_1$. La fenêtre de la moyenne glissante est toujours de durée égale à P éléments, mais les éléments nuls compris dans cette fenêtre ne sont pas pris en compte dans le calcul de la moyenne. Ainsi, il est possible de calculer un élément de la liste interpolée $LI_2$ si il existe au moins un élément non nul dans la fenêtre glissante de durée P appliquée à la liste normalisée $LI_1$. L'opération d'interpolation 102 permet de lisser les valeurs afin de supprimer les périodes sans indice, si ces périodes sont inférieures à la taille P de la fenêtre glissante.

La liste interpolée $LI_2$ obtenue est représentée sur la figure 1b, elle contient P-1 éléments supplémentaires par rapport à la liste normalisée $LI_1$. Comme la liste normalisée $LI_1$ comporte une période de durée supérieure à P (pris égal à 7 dans l'exemple de la figure 1b) pendant laquelle aucun indice n'est calculé, il en résulte que la liste interpolée $LI_2$ comporte encore plusieurs cases sans indice.

Pour résoudre ce problème et tenter d'obtenir une courbe continue, dans une variante de réalisation de l'invention, on opère 104 une seconde interpolation de même paramètre P que la première itération. Cette seconde interpolation permet également d'obtenir une liste finale $LI_3$ qui comporte le même nombre d'éléments que la liste initiale $LI_0$.

Lorsque le nombre d'éléments consécutifs sans indice, dans la liste interpolée $LI_2$, est supérieur ou égal à P (ce qui est le cas dans l'exemple de la figure 1b où P est égal à 7), on opère une étape supplémentaire de filtrage 105.

Cette étape de filtrage 105 consiste à supprimer les P-1 éléments situés avant un élément nul ainsi que les P-1 éléments situés après un élément nul. En effet, lorsque des éléments nuls subsistent après la seconde interpolation 104, cela signifie qu'un trop grand nombre d'éléments nuls consécutifs étaient présent dans la liste initiale et que par conséquent les valeurs interpolées dans cette période ne sont pas fiables. On préfère donc supprimer les valeurs calculées et conserver une période sans indices dans la liste finale au lieu d'élaborer des valeurs interpolées non fiables. Sur la figure 1b, la liste $LI_3$ contient encore un élément nul I après la seconde interpolation 104. Les 6 éléments situés avant l'élément I ainsi que les 6 éléments situés après (représentés en hachures) sont remis à zéro dans la liste finale.

L'étape de filtrage 105 permet donc de gérer les scénarii où aucun indice n'a été calculé pendant une longue période et où l'utilisateur souhaite, après cette période, à nouveau évaluer son indice de forme.

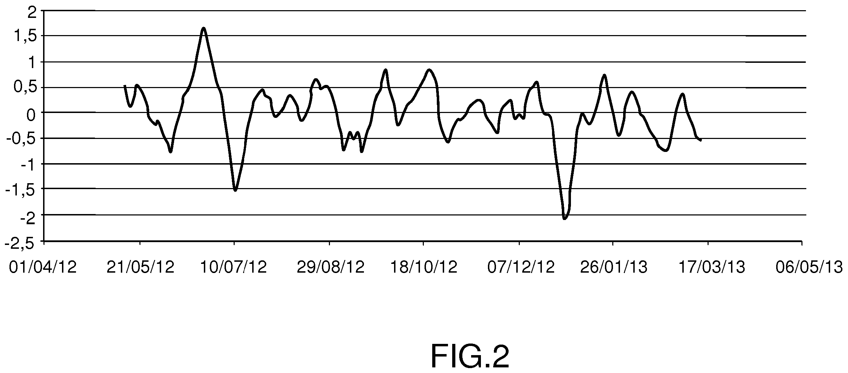

La figure 2 représente l'allure de la courbe d'évolution de l'état de forme d'un individu obtenue par application de la méthode décrite ci-dessus.

La période d'évaluation commence le 21/05/2012 et s'achève le 17/03/2013. Les valeurs de la courbe sont centrées autour de zéro. Les valeurs positives indiquent une évolution vers un état de forme tandis que les valeurs négatives indiquent une évolution vers un état de fatigue.

La génération de cette courbe de l'évolution de l'état de forme est notamment avantageuse dans le cas du suivi de l'entrainement d'un sportif de haut niveau. Le sportif peut, à n'importe quel moment, requérir l'établissement de sa courbe d'évolution d'état de forme même après une période longue sans requête. La normalisation des valeurs d'indice permet d'obtenir des valeurs cohérentes et comparables entre elles, même sur une longue durée et qui prennent en compte les caractéristiques physiques spécifiques de l'individu. Une génération de cette courbe apporte également de nombreux avantages dans le cadre d'un suivi médical (évolution d'une pathologie, réhabilitation,...) avec une mesure régulière de l'état de forme du patient.

A partir de la courbe décrite à la figure 2, il est possible d'effectuer une analyse de l'état de forme ou de fatigue d'un individu de façon automatisée. Par exemple, des valeurs statistiques peuvent être calculées automatiquement telles que la valeur la plus élevée et la valeur la plus basse sur une période de temps donnée ou encore la semaine présentant une moyenne de valeurs la plus élevée ou la plus basse.

Il est également possible de fixer des seuils de détection qui permettent d'identifier des évènements correspondant à un état de forme élevé ou à un état de fatigue élevé chez l'individu. Dans l'exemple de la figure 2, un état de fatigue élevé peut par exemple être détecté pour des valeurs d'indicateur inférieures à -1. Inversement, un état de forme élevé peut être détecté pour des valeurs d'indicateur supérieures à 1. Les seuils de détection peuvent être configurés à des valeurs différentes de 1 et -1 qui ne sont données qu'à titre d'exemples nullement limitatifs.

L'analyse de la courbe produite par la méthode selon l'invention permet à un médecin ou à un entraineur d'un sportif, de mettre en relation l'évolution de l'état de forme de l'individu avec un suivi médical ou un suivi d'entrainement.

La méthode selon l'invention, et en particulier l'étape de normalisation, permet une comparaison immédiate d'un niveau de variabilité de fréquence cardiaque à un instant donné avec un niveau de variabilité de fréquence cardiaque obtenue à un instant antérieur. L'utilisateur de la courbe produite par l'invention peut obtenir une information immédiate sur le niveau de forme à un instant donné de l'individu sans nécessité d'effectuer une comparaison avec des mesures passées.

Enfin, un avantage important de l'invention est qu'elle permet la production d'une courbe de variation de l'état de forme qui est valable pour plusieurs individus et qui peut donc être automatisée. Il en résulte un gain de temps significatif pour l'utilisateur, qui peut être un médecin ou un entraîneur de sportif, dans l'analyse de l'état de forme instantané d'un individu.

On décrit à présent plusieurs indicateurs qui peuvent être utilisés comme données d'entrées de la méthode selon l'invention décrite ci-dessus.

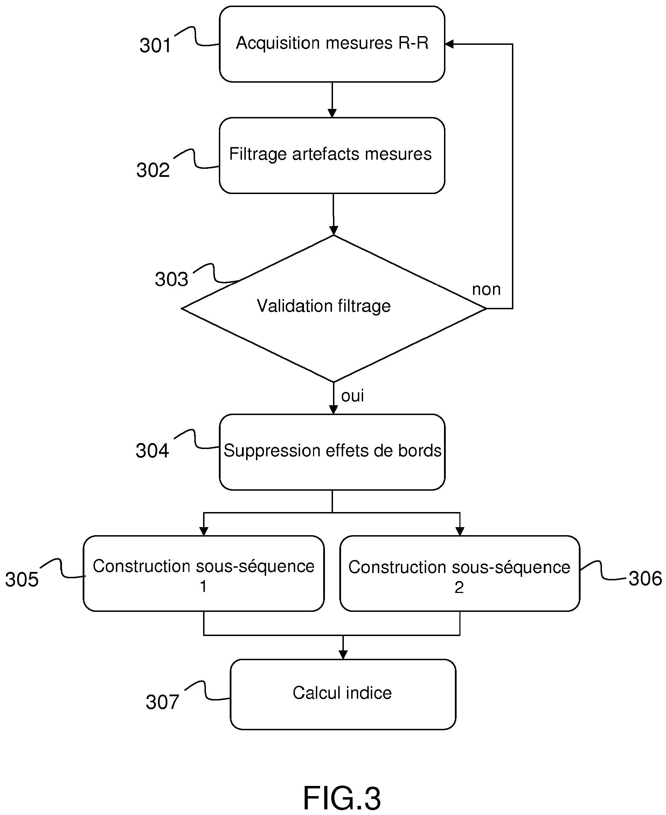

La figure 3 détaille, sur un organigramme, les étapes de mise en œuvre d'une méthode de détermination d'un indice représentatif de l'activité sympatho-vagale d'un individu. Un tel indice peut être utilisé comme indicateur représentatif de la variabilité de la fréquence cardiaque d'un individu dans la méthode d'élaboration d'une courbe de forme selon l'invention.

Selon une première étape 301 de cette méthode, on collecte une pluralité de mesures de l'intervalle de temps entre deux battements cardiaques consécutifs d'un individu. Ces mesures sont réalisées entre deux pics d'amplitude consécutifs sur l'onde R d'un électrocardiogramme qui est l'onde de plus grande amplitude et sont désignées par l'expression « mesures R-R » bien connue du domaine. Les durées successives des intervalles R-R sont inversement proportionnelles aux fréquences cardiaques instantanées de l'individu.

L'acquisition de mesures R-R peut être effectuée à l'aide de différents capteurs. Elle peut se faire par mesure sur un électrocardiogramme enregistré à l'aide d'un cardiofréquencemètre ou par le biais de tout type de capteurs permettant la mesure d'un électrocardiogramme sur un individu ou directement la mesure des intervalles R-R. De tels capteurs comprennent en particulier une ceinture thoracique cardiaque, un textile instrumenté ou des capteurs déportés sur des zones du corps éloignées du cœur ou du thorax tel des capteurs positionnés sur le lobe de l'oreille ou sur l'extrémité d'un doigt. L'acquisition des mesures R-R peut être effectuée en milieu hospitalier par un personnel médical mais peut également, grâce à certains capteurs précités, être réalisée directement par l'individu. Le capteur de mesure peut notamment être embarqué dans un téléphone intelligent ou dans un bracelet montre intelligent.

L'acquisition des mesures R-R peut être réalisée en une ou plusieurs sessions d'acquisition. La durée totale de l'acquisition doit être suffisante pour permettre l'exécution de la méthode de calcul de l'indicateur. En pratique, une durée d'acquisition au moins égale à cinq minutes est suffisante.

Les mesures R-R sont stockées dans une base de données sous la forme d'une séquence initiale. Préférentiellement, les mesures R-R sont acquises sur un individu dans une situation de repos.

Une deuxième étape 302 de la méthode permet le filtrage automatisé des artefacts de mesures dans la séquence initiale. Cette deuxième étape 302 produit en sortie une séquence de mesures R-R filtrées dans laquelle une partie des mesures sont éliminées car identifiées comme des mesures aberrantes résultant des imperfections des équipements de mesure. Un avantage lié à cette deuxième étape 302 particulière est de permettre une automatisation du filtrage des artefacts alors que cette opération doit usuellement être réalisée par un opérateur et de façon manuelle.

Une façon de réaliser le filtrage de la séquence initiale est décrite à présent en se basant sur l'utilisation de six filtres consécutifs.

La séquence de mesures R-R initialement acquise est notée séquence L0. La séquence L0 comprend une pluralité de mesures R-R consécutives indicées par l'entier i qui varie de 0 à N-1, où N est le nombre de mesures réalisées.

Un premier filtre F1 est appliqué à la séquence initiale L0 afin de supprimer les valeurs extrêmes instantanées. Une mesure est supprimée de la séquence si sa valeur est supérieure à un premier seuil ou si sa valeur est inférieure à un deuxième seuil. Le premier et le deuxième seuil sont fixés en fonction de l'amplitude maximale et minimale envisageable pour l'intervalle entre deux battements cardiaques d'un cœur humain. Par exemple, le premier seuil peut être fixé à 5000 et le deuxième seuil à 200. Ces seuils sont configurables et peuvent notamment prendre en compte les caractéristiques personnelles de l'individu, notamment son profil de santé.

L'application du premier filtre F1 permet l'obtention d'une première séquence filtrée L1. Ce premier filtre F1 a pour avantage de permettre la suppression automatisée de valeurs physiologiquement impossibles dans la séquence de mesures R-R.

Un deuxième filtre F2 peut être appliqué à la première séquence filtrée L1 afin de supprimer les valeurs extrêmes mais cette fois de façon relative. Autrement dit, le deuxième filtre F2 vise à supprimer les mesures qui ont une valeur très supérieure ou très inférieure à la valeur de la mesure précédente. Une mesure est supprimée de la séquence L1 si le rapport entre la valeur de cette mesure et la valeur de la mesure précédente est supérieur à un troisième seuil ou inférieur à un quatrième seuil. Les seuils sont configurables de la même façon que pour le premier filtre F1. Une valeur possible pour le troisième seuil est 1,75. Une valeur possible pour le quatrième seuil est 0,25. L'application du deuxième filtre F2 permet l'obtention d'une deuxième séquence filtrée L2. Ce deuxième filtre F2 a pour avantage de permettre la suppression automatisée de valeurs physiologiquement impossibles dans la séquence de mesures R-R en prenant en compte les variations d'amplitude des mesures R-R spécifiques à chaque individu. Par exemple, les variations d'amplitude sont plus élevées chez un sportif de haut niveau que chez un individu sédentaire ou chez un patient hospitalisé. L'utilisation d'un filtrage relatif permet de s'adapter automatiquement au type de profil physiologique de l'individu.

Un troisième filtre F3 peut être appliqué à la deuxième séquence filtrée L2 afin de supprimer, de façon absolue, les écarts extrêmes entre deux mesures consécutives.

Une mesure est supprimée de la séquence L2 si

- l'écart entre cette mesure et la précédente et l'écart entre cette mesure et la suivante est supérieur à un cinquième seuil, ou
- l'écart entre cette mesure et la précédente et l'écart entre cette mesure et la suivante est inférieur à un sixième seuil.

Les seuils sont configurables de la même façon que pour les filtres précédents. Une valeur possible pour le cinquième seuil est 400. Une valeur possible pour le sixième seuil est -400.

L'application du troisième filtre F3 permet l'obtention d'une troisième séquence filtrée L3. Le troisième filtre F3 a pour avantage de permettre la suppression automatisée de valeurs physiologiquement impossibles dans les écarts entre deux mesures R-R successives.

Un quatrième filtre F4 peut être appliqué à la troisième séquence filtrée L3 afin de supprimer, de façon relative, les écarts extrêmes entre deux mesures consécutives.

Une mesure est supprimée de la séquence L3 si

- le rapport entre cette mesure et la précédente et le ratio entre cette mesure et la suivante est supérieur à un septième seuil, ou
- le rapport entre cette mesure et la précédente et le ratio entre cette mesure et la suivante est inférieur à un huitième seuil.

Les seuils sont configurables de la même façon que pour les filtres précédents. Une valeur possible pour le septième seuil est 1,4. Une valeur possible pour le huitième seuil est 0,6.

L'application du quatrième filtre F4 permet l'obtention d'une quatrième séquence filtrée L4. Le quatrième filtre F4 a pour avantage de permettre la suppression automatisée de valeurs physiologiquement impossibles dans les écarts entre deux mesures R-R successives en prenant en compte les variations d'amplitude desdits écarts spécifiques à chaque individu de façon similaire à l'avantage obtenu par le deuxième filtre F2 pour la suppression des valeurs physiologiquement impossibles.

Un cinquième filtre F5 peut être appliqué à la quatrième séquence filtrée L4 afin de supprimer des valeurs extrêmes par rapport à l'écart type des valeurs calculé sur l'ensemble de la séquence.

Une mesure est supprimée de la séquence L4 si la valeur absolue de l'écart entre cette mesure et la précédente est supérieure à un neuvième seuil dépendant de l'écart type calculé sur l'ensemble des mesures de la séquence. Le neuvième seuil est configurable et peut, par exemple, être pris égal à $4\sigma$, où $\sigma$ est égal à l'écart-type calculé sur l'ensemble des mesures de la séquence filtrée L4.

L'application du cinquième filtre F5 permet l'obtention d'une quatrième séquence filtrée L5.

Un sixième filtre F6 peut être appliqué à la cinquième séquence filtrée L5 afin de supprimer des écarts extrêmes entre deux mesures consécutives, par rapport à l'écart type de l'ensemble des mesures.

Une mesure est supprimée de la séquence L6 si

- l'écart entre cette mesure et la précédente et l'écart entre cette mesure et la suivante sont supérieurs à un dixième seuil dépendant de l'écart type calculé sur l'ensemble des mesures de la séquence, ou
- l'écart entre cette mesure et la précédente et l'écart entre cette mesure et la suivante sont inférieurs à un onzième seuil dépendant de l'écart type calculé sur l'ensemble des mesures de la séquence.

Les seuils sont configurables de la même façon que pour les filtres précédents. Le dixième seuil est par exemple pris égal à $2,5\sigma$ et le onzième seuil est par exemple pris égal à $-2,5\sigma$. $\sigma$ est égal à l'écart-type calculé sur l'ensemble des mesures de la séquence filtrée L5.

Le cinquième et le sixième filtre permettent d'affiner encore d'avantage le filtrage des artefacts de mesure. Les seuils de filtrage des quatre premiers filtres sont configurés de sorte à laisser passer une partie des artefacts de mesure d'amplitude faible mais de façon à ne pas filtrer ou à limiter le filtrage de mesures contenant une information réelle.

L'application du cinquième filtre et du sixième filtre permet de supprimer le bruit résiduel restant après l'application des quatre premiers filtres.

L'application successive des six filtres décrit ci-dessus a pour avantage un filtrage progressif du bruit de mesure et permet de minimiser la probabilité de filtrage de mesures contenant de l'information.

Sans sortir du cadre de l'invention, les étapes de filtrage décrites ci-dessus peuvent être réalisées par des filtres différents pour obtenir le même résultat. Par exemple, les six étapes de filtrage peuvent être réalisées à l'aide d'un filtre unique configuré pour appliquer directement un ou plusieurs critères de filtrage des mesures ou des écarts en fonction de leur valeur.

L'application successive des six filtres décrits ci-dessus dans l'ordre indiqué présente des avantages particuliers qui ont été explicités. Cependant, la méthode n'est pas limitée à cette forme particulière de réalisation du filtrage. En particulier, la méthode peut être réalisée en appliquant seulement une partie des filtres, par exemple uniquement le premier filtre ou uniquement les N premiers filtres avec N un nombre entier strictement inférieur à 6.

La fonction de l'étape de filtrage 302 est de supprimer les artefacts de mesure éventuels qui peuvent apparaitre du fait de l'imperfection des capteurs utilisés. L'étape de filtrage 302 prend en compte la connaissance de l'évolution des battements cardiaques chez l'être humain et en particulier les valeurs extrêmes possibles pour les mesures R-R.

Si les capteurs de mesures R-R utilisés permettent de fournir des mesures sans, ou avec un nombre limité, d'artefacts, il est également envisageable que l'étape de filtrage 302 soit optionnelle.

A l'issu de l'ensemble des étapes de filtrage, on obtient une séquence filtrée finale.

L'ensemble des seuils de filtrage peuvent être configurés conjointement ou indépendamment les uns des autres en fonction de caractéristiques présupposées de l'évolution du battement cardiaque d'un être humain. Les seuils de filtrage sont adaptatifs et sont notamment configurés pour minimiser la probabilité de filtrer une mesure contenant de l'information. Les seuils peuvent être également configurés en prenant en compte des caractéristiques personnelles de l'individu, par exemple le fait que l'individu soit un sportif de haut niveau.

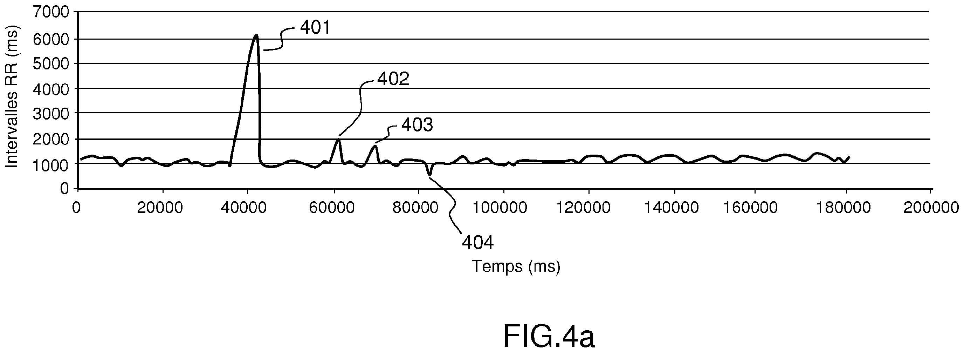

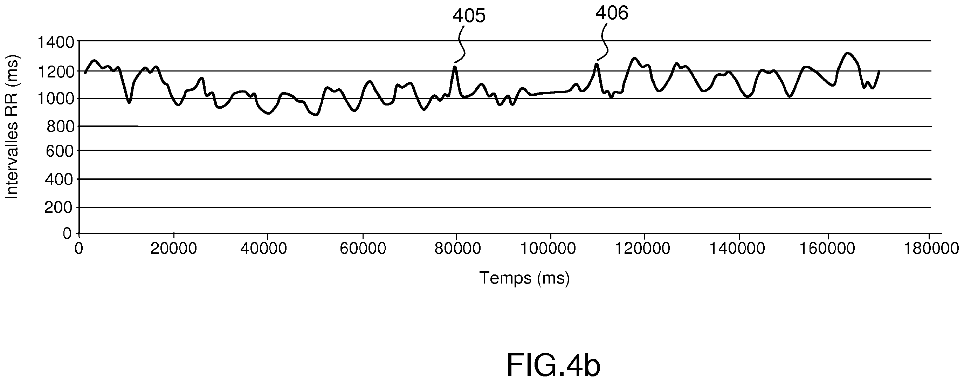

Les figures 4a et 4b illustrent, sur deux diagrammes, le profil des mesures R-R sur la durée d'une séquence à deux stades du procédé.

La figure 4a représente un exemple de séquence initiale L0 de mesures R-R acquises pour un individu donné. Les références 401,402,403,404 identifient sur la courbe de mesure différents artefacts de mesure.

La mesure 401 correspond à une mesure dont la valeur est très élevée et peut être filtrée par l'application du premier filtre F1.

La mesure 402 présente une valeur élevée relativement à la mesure précédente et peut être filtrée par l'application du deuxième filtre F2.

La mesure 403 présente un écart absolu important par rapport à la mesure précédente et à la mesure suivante et peut être filtrée par l'application du troisième filtre F3.

La mesure 404 présente un écart relatif important par rapport à la mesure précédente et à la mesure suivante et peut être filtrée par l'application du quatrième filtre F4.

La figure 4b représente la séquence filtrée obtenue à l'issue de l'application successive des quatre premiers filtres F1,F2,F3,F4.

Les artefacts les plus significatifs ont été supprimés, cependant certaines mesures aberrantes peuvent encore être effacées. La mesure 405 correspond à une valeur extrême par rapport à l'écart type de l'ensemble des mesures et peut être supprimé par application du cinquième filtre F5. La mesure 406 correspond à un écart extrême par rapport à l'écart type de l'ensemble des mesures et peut être supprimé par application du sixième filtre F6.

Une troisième étape 303 de validation du filtrage est ensuite opérée afin de vérifier que la séquence filtrée obtenue contient suffisamment de mesures pour pouvoir calculer un indicateur fiable. La troisième étape 303 consiste à tester que le nombre de mesures de la séquence filtrée finale est au moins égal à un nombre minimum de mesures ou à un pourcentage du nombre de mesures présentes dans la séquence initiale L0. Par exemple, le test selon la troisième étape 303 peut consister à vérifier que la séquence filtrée contient au moins 90% des éléments de la séquence initiale L0.

Si le nombre de mesures contenues dans la séquence filtrée finale est jugé trop faible, la phase d'acquisition de la méthode est inexploitable et on effectue alors une nouvelle étape 301 d'acquisition de mesures R-R.

Selon une quatrième étape 304 de la méthode, les effets de bords affectant les mesures R-R acquises sont corrigés.

Lors d'une séance d'acquisition de mesures, le début de la séquence acquise peut être perturbé car il faut un temps minimum pour obtenir une stationnarité dans les mesures cardiaques réalisées. De même, la fin de la séquence acquise peut également être perturbée si, par exemple, l'individu se relève ou effectue des gestes brusques. Les effets de bord peuvent induire des dérives linéaires progressives qui ne sont pas détectées par l'étape de filtrage 302 qui vise principalement à éliminer des ruptures de continuité brutales.

L'étape 304 consiste à supprimer un nombre de mesures prédéterminé au début et à la fin de la séquence. Ce nombre peut correspondre à une durée prédéterminée, par exemple une durée égale à 30 secondes. La durée de la portion supprimée au début de la séquence correspond au temps nécessaire pour arriver à un état stationnaire. La durée de la portion supprimée à la fin de la séquence correspond au temps s'écoulant entre la fin de l'état stationnaire, qui peut être initiée par un mouvement de l'individu, et la fin de l'enregistrement.

Optionnellement, l'étape 304 peut être éliminée si le capteur de mesure utilisé permet d'obtenir directement une séquence de mesures correspondant totalement à un état stationnaire du rythme cardiaque, par exemple, si le début et la fin de l'enregistrement peuvent être automatisés sans intervention de l'individu.

A partir de la séquence de mesures R-R filtrée obtenue à l'issue des étapes précédentes, on construit ensuite, dans une cinquième étape 305, une sous-séquence composée des écarts positifs entre deux valeurs consécutives de la séquence initiale. Autrement dit, si on note $\{RR_0,... RR_i ,..., RR_{N-1}\}$ les mesures de la séquence obtenue en sortie de l'étape 104, et $\{k_0 = RR_1 - RR_0 ; k_1 = RR_2 - RR_1 ;... ; k_n = RR_{n+1} - RR_n\}$ les écarts positifs entre deux valeurs consécutives, on construit une sous-séquence composée des valeurs $k_i$ positives.

Dans une sixième étape 306, on construit également une deuxième sous-séquence composée cette fois des valeurs absolue des écarts négatifs entre deux valeurs consécutives. Cette deuxième sous-séquence comprend les valeurs absolues des valeurs $k_i$ négatives.

Enfin, dans une dernière étape 307, on détermine l'indice représentatif de l'activité sympatho-vagale de l'individu à partir des valeurs des deux sous-séquences déterminées aux étapes 305 et 306. Plus précisément, l'indice déterminé à l'étape 307 est calculé comme une information représentative de la comparaison entre une première valeur statistique représentative des valeurs de la première sous-séquence et une seconde valeur statistique représentative des valeurs de la seconde sous-séquence.

Par exemple, l'indice peut être calculé comme le ratio entre la moyenne des valeurs de la seconde sous-séquence et la moyenne des valeurs de la première sous-séquence :

$$I = moy(SS2)/moy(SS1) \quad (1)$$

L'indice peut également être calculé en représentation logarithmique : $I = \ln(moy(SS2)/moy(SS1))*10$, où ln désigne la fonction logarithme népérien. Mais l'indice peut également être égal au ratio entre l'écart-type des valeurs de la seconde sous-séquence et l'écart type des valeurs de la première sous-séquence :

$$I = écart\text{-}type(SS2)/ \ écart\text{-}type(SS1) \quad (2)$$

L'objectif du calcul de l'indice est d'obtenir une information représentative de la comparaison entre les valeurs de la première sous-séquence (écarts positifs) qui représentent la proportion de l'influence du système nerveux parasympathique et les valeurs de la seconde sous-séquence (valeur absolue des écarts négatifs) qui représentent la proportion de l'influence du système nerveux sympathique. Par exemple, si l'indice est donné par la formule (1) ou la formule (2), une valeur élevée de l'indice correspond à une prépondérance du système sympathique tandis qu'une valeur faible correspond à une prépondérance du système parasympathique. Une valeur de l'indice proche de la valeur 1 correspond à une situation équilibrée entre l'influence des deux systèmes.

De façon générale, l'indice calculé à l'étape 307 peut être formulé comme le ratio entre une fonction de la première sous-séquence et une fonction de la seconde sous-séquence. Bien entendu, le ratio inverse peut également être utilisé comme indice.

L'indice ainsi calculé par la méthode décrite ci dessus donne une information fiable sur l'état de forme de l'individu. En particulier, dans le cas d'un sportif de haut niveau, l'indice calculé permet, à partir d'une méthode d'analyse temporelle, de caractériser à la fois l'influence de l'activité sympathique et l'influence de l'activité parasympathique sans être dépendante de la respiration de l'individu.

La méthode d'élaboration d'une courbe de l'état de forme d'un individu n'est pas limitée à une mise en œuvre avec l'indice représentatif de l'activité sympatho-vagale obtenu par la méthode décrite ci-dessus. Tout autre indice représentatif de la variabilité de la fréquence cardiaque d'un individu peut être utilisé pour élaborer une courbe de suivi de l'évolution de l'état de forme de l'individu.

En particulier, des indicateurs obtenus via des méthodes d'analyse dans le domaine temporel ou dans le domaine non-linéaire ou dans le domaine fréquentiel appliquées à une pluralité de mesures R-R sont compatibles de l'invention.

De tels indicateurs sont par exemple décrits dans le document [6].

Des indicateurs obtenus par analyse dans le domaine temporel comprennent notamment la fréquence cardiaque moyenne, un indicateur de variabilité globale du type SDNN de l'acronyme anglais « Standard Deviation of the NN inverval », un indicateur de variabilité court terme parasympathique du type RMSSD égal à la racine carrée de la moyenne au carré des écarts successifs entre intervalles R-R adjacents ou du type pNN50 égal au pourcentage d'intervalles RR successivement différents.

Des indicateurs obtenus par analyse dans le domaine non linéaire comprennent notamment un indicateur de variabilité court terme parasympathique du type SD1 ou de variabilité globale du type SD2 tels que décrits dans le document [5].

Des indicateurs obtenus par analyse dans le domaine fréquentiel comprennent notamment la densité spectrale de puissance du spectre fréquentiel de la répartition des intervalles R-R mesuré dans toute la bande de fréquence ou dans une sous-bande fréquentielle très basses fréquences, basses fréquences ou hautes fréquences ou encore un indicateur de la balance sympatho-vagale entre les basses fréquences et les hautes fréquences.

La méthode d'élaboration d'une courbe de l'état de forme peut être implémentée à partir d'éléments matériel et/ou logiciel. Elle peut notamment être mise en œuvre en tant que programme d'ordinateur comportant des instructions pour son exécution. Le programme d'ordinateur peut être enregistré sur un support d'enregistrement lisible par un processeur. Il peut être exécuté par un serveur sécurisé ou directement sur un terminal. La courbe peut être générée sur requête d'un utilisateur. La requête peut être transmise au serveur sécurisé via un terminal qui peut être un téléphone intelligent, une tablette numérique, un ordinateur portable ou tout autre équipement électronique apte à communiquer sans fils avec un serveur à distance. La courbe générée peut être transmise par le serveur vers le terminal puis affichée sur l'écran du terminal.

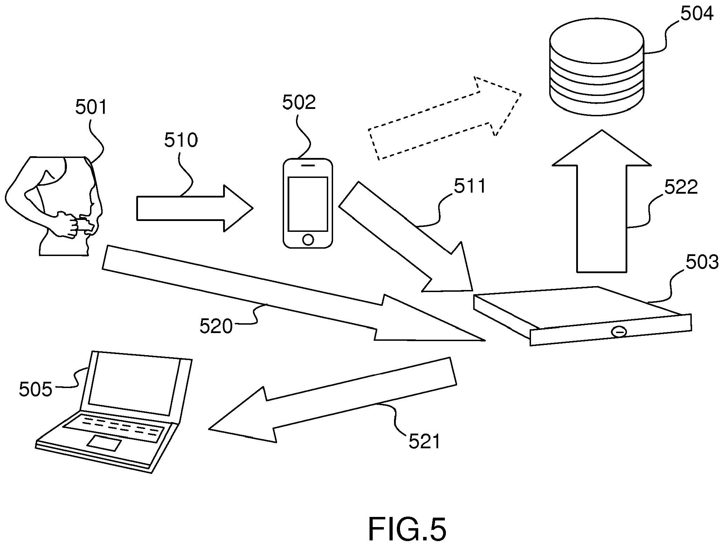

La figure 5 illustre, sur un schéma, une mise en œuvre possible de l'invention.

L'acquisition des mesures R-R est réalisée à l'aide d'un capteur 501 de mesure qui peut être un cardiofréquencemètre ou un textile instrumenté ou un autre capteur embarqué.

Les mesures R-R enregistrées peuvent être transmises 510 du capteur 501 vers un téléphone intelligent 502 ou un terminal du type tablette électronique ou ordinateur portatif ou tout autre dispositif électronique apte à communiquer avec le capteur de mesure 501.

La séquence enregistrée par le terminal 502 peut ensuite être envoyée 511 vers un serveur sécurisé 503 si l'on ne souhaite pas que les données acquises soient accessibles de façon non sécurisée. Le serveur sécurisé 503 peut être associé à une base de données 504 pour stocker les différentes données nécessaires à l'exécution de la méthode selon l'invention.

L'utilisateur 501 peut, sur requête, interroger 520 le serveur sécurisé 503 pour demander l'établissement de sa courbe d'évolution d'état de forme sur une période de temps spécifiée.

Le serveur 503 interroge ensuite la base de données 504 pour obtenir l'ensemble des indicateurs stockés sur la période de temps demandée. Le serveur 503 exécute la méthode selon l'invention pour produire la courbe de forme et la transmet 521 vers un terminal 505 pour un affichage sur l'écran de ce terminal pour que l'utilisateur 501 puisse visualiser sa courbe de forme.

La méthode selon l'invention peut être implémentée à partir d'éléments matériel et/ou logiciel. Elle peut notamment être mise en œuvre en tant que programme d'ordinateur comportant des instructions pour son exécution. Le programme d'ordinateur peut être enregistré sur un support d'enregistrement lisible par un processeur. Le support peut être électronique, magnétique, optique ou électromagnétique. Le programme d'ordinateur peut être exécuté par le serveur sécurisé 503. Le serveur sécurisé peut utiliser, pour mettre en œuvre l'invention, un ou plusieurs circuits électroniques dédiés ou un circuit à usage général. La technique de l'invention peut se réaliser sur une machine de calcul reprogrammable (un processeur ou un micro contrôleur par exemple) exécutant un programme comprenant une séquence d'instructions, ou sur une machine de calcul dédiée (par exemple un ensemble de portes logiques comme un FPGA ou un ASIC, ou tout autre module matériel).

A titre d'exemple d'architecture matérielle adaptée à mettre en œuvre l'invention, le serveur sécurisé 503 peut comporter un bus de communication auquel sont reliés une unité centrale de traitement ou microprocesseur (CPU, acronyme de « *Central Processing Unit »* en anglais; une mémoire morte (ROM, acronyme de *« Read Only Memory »* en anglais) pouvant comporter les programmes nécessaires à la mise en œuvre de l'invention; une mémoire vive ou mémoire cache (RAM, acronyme de *« Random Access Memory »* en anglais) comportant des registres adaptés à enregistrer des variables et paramètres créés et modifiés au cours de l'exécution des programmes précités ; et une interface de communication ou E/S (I/O acronyme de *« Input/ouput »* en anglais) adaptée à transmettre et à recevoir des données.

Le code logiciel selon l'invention peut être exécuté sur n'importe quel processeur approprié (par exemple, un microprocesseur) ou cœur de processeur ou un ensemble de processeurs, qu'ils soient prévus dans un dispositif de calcul unique ou répartis entre plusieurs dispositifs de calcul (par exemple tels qu'éventuellement accessibles dans l'environnement du dispositif). Le code exécutable de chaque programme permettant au dispositif programmable de mettre en œuvre les processus selon l'invention, peut être stocké, par exemple, dans le disque dur ou en mémoire morte. De manière générale, le ou les programmes pourront être chargés dans un des moyens de stockage du dispositif avant d'être exécutés. L'unité centrale peut commander et diriger l'exécution des instructions ou portions de code logiciel du ou des programmes selon l'invention, instructions qui sont stockées dans le disque dur ou dans la mémoire morte ou bien dans les autres éléments de stockage précités.

Le programme d'ordinateur selon l'invention peut être stocké dans ou sur un médium de stockage amovible (par exemple une carte SD, un DVD ou Bluray, un moyen de stockage de masse tel qu'un disque dur e.g. un SSD) ou non-amovible, volatile ou non-volatile, ce médium de stockage étant lisible partiellement ou totalement par un ordinateur ou un processeur. Le support lisible par ordinateur peut être transportable ou communicable ou mobile ou transmissible (i.e. par un réseau de télécommunication 2G, 3G, 4G, Wifi, fibre optique ou autre).

La référence à un programme d'ordinateur qui, lorsqu'il est exécuté, effectue l'une quelconque des fonctions décrites précédemment, ne se limite pas à un programme d'application s'exécutant sur un ordinateur hôte unique. Au contraire, les termes programme d'ordinateur et logiciel sont utilisés ici dans un sens général pour faire référence à tout type de code informatique (par exemple, un logiciel d'application, un micro logiciel, un microcode, ou toute autre forme d'instruction d'ordinateur) qui peut être utilisé pour programmer un ou plusieurs processeurs pour mettre en œuvre des aspects des techniques décrites ici. Les moyens ou ressources informatiques peuvent notamment être distribués ("*Cloud computing*"), éventuellement avec selon des technologies de pair-à-pair.

La courbe de l'état de forme générée selon l'invention présente l'avantage de pouvoir fournir une information fiable et rapide sur l'état de forme d'un individu. Elle présente notamment un intérêt pour un entraineur d'un sportif de haut niveau qui peut utiliser cette courbe afin d'améliorer l'entrainement du sportif.

Des statistiques peuvent être élaborées à partir de la courbe de l'état de forme, en particulier, la valeur la plus élevée et la plus faible sur une période donnée ou encore la moyenne la plus élevée et la plus faible sur une sous-période, par exemple égale à une semaine, la sous-période pouvant correspondre à un cycle d'entrainement.

Des seuils de détection d'événements anormaux peuvent également élaborés, par exemple un seuil de valeur d'indice de forme en dessous duquel l'état de fatigue du sportif est tel qu'il y a risque de blessures.

Sans sortir du cadre de l'invention, celle-ci s'applique également à la surveillance de l'état de forme d'un animal de compétition, notamment dans le cadre de disciplines sportives animales. L'animal de compétition peut être un animal de course, par exemple un cheval de course, un chien de course ou un chameau ou dromadaire de course. L'invention peut également s'étendre à d'autres disciplines sportives animales ou compétitions animales, notamment équestres, tels que le dressage, le saut d'obstacles ou le parcours, pour les chevaux.

La méthode de détermination d'un indice représentatif de l'activité sympatho-vagale d'un individu telle que décrite à la figure 3 peut s'appliquer à l'identique pour un animal de compétition. Les différents seuils utilisés dans les différentes étapes de la méthode décrite peuvent être adaptés ou réévalués en fonction du niveau d'entrainement de l'animal.

## Références

[1] "Heart rate variability. Standards of measurement, physiological interprétation and clinical use", American Heart Association Inc.: European Society of Cardiology, 1996.

[2] "The impact of breathing on HRV measurements: Implications for the longitudinal follow-up of athletes", Damien Saboul et al, European Journal of Sport Science, 2013.

[3] "Training adaptation and heart rate variability in elite endurance athletes: opening the door to effective monitoring", Daniel J. Plews, Springer International Publishing Switzerland 2013.

[4] "Conversion from vagal to sympathetic prédominance with strenuous training in high performance world class athletes", Ferdinando Iellamo, American Heart Association, 2002

[5] " Correlations between the Poincaré Plot and conventional heart rate variability parameters assessed during paced breathing", Guzik et al, J.Physiol. Sci. vol 57, n°1, feb 2007, pp 63-71.

[6] " Heart rate variability in athletes", Aubert et al. Sports Med 2003.

## Revendications

**1.** Méthode, mise en œuvre par ordinateur, d'élaboration automatisée d'une courbe d'évolution d'un indicateur représentatif de la variabilité de la fréquence cardiaque d'un individu ou d'un animal de compétition, ladite méthode comprenant les étapes suivantes :

- Détermination (101) d'une séquence d'une pluralité de mesures d'un indicateur représentatif de la variabilité de la fréquence cardiaque, lesdites mesures étant réalisées avec une période temporelle constante prédéterminée, au moins une occurrence parmi lesdites mesures pouvant être omise, ladite séquence comprenant, pour chaque occurrence temporelle de ladite période, soit une mesure soit une valeur nulle correspondant à une absence de mesure à l'occurrence temporelle associée,
- Normaliser (102) les mesures de ladite séquence en excluant de la normalisation les valeurs nulles,
- Effectuer au moins une interpolation (103,104) de la séquence normalisée en effectuant une moyenne glissante sur une durée P prédéterminée des valeurs de la séquence, les valeurs nulles étant exclues de la moyenne,
- Si au moins une valeur nulle, correspondant à une mesure absente, subsiste dans la séquence interpolée, filtrer (105) ladite séquence en annulant les P-1 valeurs précédant ladite au moins une valeur nulle et les P-1 valeurs suivant ladite au moins une valeur nulle.

**2.** Méthode d'élaboration automatisée d'une courbe d'évolution d'un indicateur selon la revendication 1 comprenant deux interpolations (103,104) successives de la séquence normalisée.

**3.** Méthode d'élaboration automatisée d'une courbe d'évolution d'un indicateur selon l'une des revendications 1 ou 2 dans laquelle ledit indicateur est un indice représentatif de l'activité sympatho-vagale d'un individu ou d'un animal de compétition obtenu en exécutant les étapes suivantes :

- Recevoir (301) une séquence comportant une pluralité de mesures, dites mesures R-R, de l'intervalle de temps entre deux battements cardiaques consécutifs de l'individu ou de l'animal de compétition,
- Construire (305) une première sous-séquence constituée des écarts positifs entre deux valeurs consécutives de la dite séquence reçue,
- Construire (306) une seconde sous-séquence constituée des valeurs absolues des écarts négatifs entre deux valeurs consécutives de la dite séquence reçue,
- Calculer (307) un indice égal à une valeur représentative de la comparaison entre le résultat d'une fonction statistique appliquée aux valeurs de la première sous-séquence et le résultat de ladite fonction statistique appliquée aux valeurs de la seconde sous-séquence.

**4.** Méthode d'élaboration automatisée d'une courbe d'évolution d'un indicateur selon la revendication 3 dans laquelle l'indice calculé est égal au ratio entre une fonction statistique appliquée aux valeurs de la première sous-séquence et ladite fonction statistique appliquée aux valeurs de la seconde sous-séquence.

**5.** Méthode d'élaboration automatisée d'une courbe d'évolution d'un indicateur selon la revendication 4 dans laquelle ladite fonction statistique est prise dans l'ensemble suivant : une moyenne, une variance, un écart type.

6. Méthode d'élaboration automatisée d'une courbe d'évolution d'un indicateur selon l'une des revendications 1 ou 2 dans laquelle ledit indicateur est égal à une mesure de la fréquence cardiaque moyenne de l'individu ou de l'animal de compétition ou à une mesure statistique déterminée à partir d'une pluralité de mesures de l'intervalle de temps entre deux battements cardiaques consécutifs de l'individu ou de l'animal de compétition.

7. Méthode d'élaboration automatisée d'une courbe d'évolution d'un indicateur selon l'une des revendications 1 ou 2 dans laquelle ledit indicateur est égal à une mesure, dans au moins une sous-bande fréquentielle, de la densité spectrale de puissance d'une pluralité de mesures de l'intervalle de temps entre deux battements cardiaques consécutifs de l'individu ou de l'animal de compétition..

8. Méthode d'élaboration automatisée d'une courbe d'évolution d'un indicateur selon l'une des revendications précédentes comprenant en outre :

   - La transmission d'une requête d'établissement de ladite courbe sur une période de temps donnée par un individu,
   - L'affichage de ladite courbe sur un écran.

9. Méthode d'élaboration automatisée d'une courbe d'évolution d'un indicateur selon l'une des revendications précédentes dans laquelle l'animal de compétition est un cheval.

10. Utilisation d'une courbe d'évolution d'un indicateur représentatif de la variabilité de la fréquence cardiaque d'un individu ou d'un animal de compétition obtenue par exécution de la méthode selon l'une des revendications 1 à 9 comprenant la configuration d'au moins un seuil de détection d'un état de l'individu ou de l'animal de compétition parmi un état de forme ou un état de fatigue et l'application dudit au moins un seuil à ladite courbe pour détecter ledit état.

11. Programme d'ordinateur comportant des instructions pour l'exécution de la méthode d'élaboration automatisée d'une courbe d'évolution d'un indicateur représentatif de la variabilité de la fréquence cardiaque d'un individu ou d'un animal de compétition selon l'une quelconque des revendications 1 à 9, lorsque le programme est exécuté par un processeur.

12. Support d'enregistrement lisible par un processeur sur lequel est enregistré un programme d'ordinateur selon la revendication 11.

13. Dispositif (503) comprenant une mémoire et un processeur configurés pour mettre en œuvre la méthode d'élaboration automatisée d'une courbe d'évolution d'un indicateur représentatif de la variabilité de la fréquence cardiaque d'un individu ou d'un animal de compétition selon l'une quelconque des revendications 1 à 9.

14. Dispositif selon la revendication 13 comprenant en outre :

   - Des moyens pour recevoir une requête d'établissement de ladite courbe sur une période de temps donnée par un individu,
   - Des moyens pour transmettre ladite courbe vers un écran d'affichage.

**Patentansprüche**

1. Computerimplementiertes Verfahren zur automatisierten Erzeugung einer Verlaufskurve eines Indikators, welcher die Herzfrequenzvariabilität eines Individuums oder eines Wettkampfstieres darstellt, wobei das Verfahren folgende Schritte umfasst:

   - Bestimmen (101) einer Sequenz einer Vielzahl von Messungen eines Indikators, welcher die Herzfrequenzvariabilität darstellt, wobei die Messungen mit einer vorbestimmten konstanten Zeitperiode vorgenommen werden, wobei mindestens ein Exemplar unter den Messungen ausgelassen werden kann, wobei die Sequenz für jedes zeitabhängige Auftreten der Periode entweder eine Messung oder einen Null-Wert umfasst, welcher einer fehlenden Messung zum zugeordneten zeitabhängigen Auftreten entspricht,
   - Normalisieren (102) der Messungen der Sequenz, indem die Null-Werte aus der Normalisierung ausgeschlossen werden,
   - Durchführen mindestens einer Interpolation (103,104) der normalisierten Sequenz, indem ein gleitender Mittelwert über eine vorbestimmte Dauer P der Werte der Sequenz durchgeführt wird, wobei die Null-Werte aus dem Mittelwert ausgeschlossen werden,
   - wenn mindestens ein Null-Wert, welcher eine fehlende Messung entspricht, in der interpolierten Sequenz verbleibt, Filtern (105) der Sequenz, indem die P-1 dem mindestens einen Null-Wert vorausgehenden Werte und die P-1 dem mindestens einen Null-Wert folgenden Werte annulliert werden.

2. Verfahren zur automatisierten Erzeugung einer Verlaufskurve eines Indikators nach Anspruch 1, umfassend zwei aufeinanderfolgende Interpolationen (103,104) der normalisierten Sequenz.

3. Verfahren zur automatisierten Erzeugung einer Verlaufskurve eines Indikators nach einem der Ansprüche 1 oder 2, wobei der Indikator ein Index ist, welcher die sympathovagale Aktivität eines Individuums oder eines Wettkampfstieres darstellt, welcher durch Ausführen der folgenden Schritte erzielt wird:

- Empfangen (301) einer Sequenz, welche eine Vielzahl von Messungen umfasst, genannt R-R-Messungen, des Zeitintervalls zwischen zwei aufeinanderfolgenden Herzschlägen des Individuums oder des Wettkampfstieres,
- Bilden (305) einer ersten Teilsequenz, bestehend aus den positiven Abweichungen zwischen zwei aufeinanderfolgenden Werten der empfangenen Sequenz,
- Bilden (306) einer zweiten Teilsequenz, bestehend aus den Absolutwerten der negativen Abweichungen zwischen zwei aufeinanderfolgenden Werten der empfangenen Sequenz,
- Berechnen (307) eines Indices, welcher einem Wert gleich ist, welcher den Vergleich zwischen dem Ergebnis einer auf die Werte der ersten Teilsequenz angewandten statistischen Funktion und dem Ergebnis der auf die Werte der zweiten Teilsequenz angewandten statistischen Funktion darstellt.

4. Verfahren zur automatisierten Erzeugung einer Verlaufskurve eines Indikators nach Anspruch 3, wobei der berechnete Index gleich dem Verhältnis zwischen einer auf die Werte der ersten Teilsequenz angewandten statistischen Funktion und der auf die Werte der zweiten Teilsequenz angewandten statistischen Funktion ist.

5. Verfahren zur automatisierten Erzeugung einer Verlaufskurve eines Indikators nach Anspruch 4, wobei die statistische Funktion aus folgender Menge gewählt ist: einem Mittelwert, einer Varianz, und einer Standardabweichung.

6. Verfahren zur automatisierten Erzeugung einer Verlaufskurve eines Indikators nach einem der Ansprüche 1 oder 2, wobei der Indikator gleich einer Messung der mittleren Herzfrequenz des Individuums oder des Wettkampfstieres oder gleich einer statistischen Messung ist, welche anhand einer Vielzahl von Messungen des Zeitintervalls zwischen zwei aufeinanderfolgenden Herzschlägen des Individuums oder des Wettkampfstieres bestimmt wird.

7. Verfahren zur automatisierten Erzeugung einer Verlaufskurve eines Indikators nach einem der Ansprüche 1 oder 2, wobei der Indikator gleich einer Messung, in mindestens einem Frequenz-Teilband, der spektralen Leistungsdichte einer Vielzahl von Messungen des Zeitintervalls zwischen zwei aufeinanderfolgenden Herzschlägen des Individuums oder des Wettkampfstieres ist.

8. Verfahren zur automatisierten Erzeugung einer Verlaufskurve eines Indikators nach einem der vorhergehenden Ansprüche, ferner Folgendes umfassend:

- die Übertragung einer Anforderung zur Erzeugung der Kurve über eine durch ein Individuum gegebenen Zeitperiode,
- die Anzeige der Kurve an einem Bildschirm.

9. Verfahren zur automatisierten Erzeugung einer Verlaufskurve eines Indikators nach einem der vorhergehenden Ansprüche, wobei das Wettkampfstier ein Pferd ist.

10. Verwendung einer Verlaufskurve eines Indikators, welcher die Herzfrequenzvariabilität eines Individuums oder eines Wettkampfstieres darstellt, erzielt durch Ausführen des Verfahrens nach einem der Ansprüche 1 bis 9, umfassen die Konfiguration mindestens eines Erkennungsschwellenwertes eines Zustandes des Individuums oder des Wettkampfstieres aus einem Zustand der Fitness oder einem Zustand der Ermüdung, und Anwendung des mindestens einen Schwellenwertes auf die Kurve zur Erkennung des Zustandes.

11. Computerprogramm, aufweisend Anweisungen zur Ausführung des Verfahrens zur automatisierten Erzeugung einer Verlaufskurve eines Indikators, welcher die Herzfrequenzvariabilität eines Individuums oder eines Wettkampfstieres darstellt, nach einem der Ansprüche 1 bis 9, wenn das Programm von einem Prozessor ausgeführt wird.

12. Prozessorlesbarer Aufzeichnungsträger, auf welchem ein Computerprogramm nach Anspruch 11 aufgezeichnet ist.

13. Vorrichtung (503), umfassend einen Speicher und einen Prozessor, welche konfiguriert sind, um das Verfahren zur automatisierten Erzeugung einer Verlaufskurve eines Indikators, welcher die Herzfrequenzvariabilität eines Individuums oder eines Wettkampfstieres darstellt, nach einem der Ansprüche 1 bis 9 zu implementieren.

14. Vorrichtung nach Anspruch 13, ferner Folgendes umfassend:

- Mittel zum Empfangen einer Anforderung zur Erzeugung der Kurve über eine durch ein Individuum gegebene Zeitperiode,
- Mittel zum Übertragen der Kurve an einem Anzeigebildschirm.

## Claims

1. A computer-implemented method for automated production of a variation curve of an indicator representing the heart rate variability of an individual or of a competition animal, said method comprising the following steps:

   - determining (101) a sequence of a plurality of measurements of an indicator representing the heart rate variability, said measurements being carried out with a predetermined constant time period, wherein at least one occurrence among said measurements can be omitted, said sequence comprising, for each time occurrence of said period, either a measurement or a zero value corresponding to a lack of measurement at the associated time occurrence;
   - normalising (102) the measurements of said sequence by excluding the zero values from the normalisation;
   - performing at least one interpolation (103, 104) of the normalised sequence by performing a sliding average over a predetermined duration P of the values of the sequence, the zero values being excluded from the average;
   - filtering (105), if at least one zero value, corresponding to a missing measurement, remains in the interpolated sequence, said sequence by cancelling the P-1 values preceding said at least one zero value and the P-1 values following said at least one zero value.

2. The method for automated production of a variation curve of an indicator according to claim 1, comprising two successive interpolations (103, 104) of the normalised sequence.

3. The method for automated production of a variation curve of an indicator according to one of claims 1 or 2, wherein said indicator is an index representing the sympathovagal activity of an individual or of a competition animal obtained by executing the following steps:

   - receiving (301) a sequence comprising a plurality of measurements, called R-R measurements, of the time interval between two consecutive heartbeats of the individual or of the competition animal;
   - constructing (305) a first sub-sequence made up of the positive deviations between two consecutive values of said received sequence;
   - constructing (306) a second sub-sequence made up of the absolute values of the negative deviations between two consecutive values of said received sequence;
   - computing (307) an index equal to a value representing the comparison between the result of a statistical function applied to the values of the first sub-sequence and the result of said statistical function applied to the values of the second sub-sequence.

4. The method for automated production of a variation curve of an indicator according to claim 3, wherein the computed index is equal to the ratio between a statistical function applied to the values of the first sub-sequence and said statistical function applied to the values of the second sub-sequence.

5. The method for automated production of a variation curve of an indicator according to claim 4, wherein said statistical function is taken from the following set: an average, a variance, a standard deviation.

6. The method for automated production of a variation curve of an indicator according to one of claims 1 or 2, wherein said indicator is equal to a measurement of the average heart rate of the individual or of the competition animal or to a statistical measurement determined on the basis of a plurality of measurements of the time interval between two consecutive heartbeats of the individual or of the competition animal.

7. The method for automated production of a variation curve of an indicator according to one of claims 1 or 2, wherein said indicator is equal to a measurement, in at least one frequency sub-band, of the power spectral density of a plurality of measurements of the time interval between two consecutive heartbeats of the individual or of the competition animal.

8. The method for automated production of a variation curve of an indicator according to one of the preceding claims, further comprising:

   - transmitting a request to establish said curve over a time period given by an individual;
   - displaying said curve on a screen.

9. The method for automated production of a variation curve of an indicator according to one of the preceding claims, wherein the competition animal is a horse.

10. Use of a variation curve of an indicator representing the heart rate variability of an individual or of a competition animal obtained by executing the method according to one of claims 1 to 9, comprising the configuration of at least one detection threshold of a state of the individual or of the competition animal among a state of form or a state of fatigue and the application of said at least one threshold to said curve for detecting said state.

11. A computer program with instructions for executing the method for automated production of a variation curve of an indicator representing the heart rate variability of an individual or of a competition animal according to one of claims 1 to 9, when the program is executed by a processor.

12. A processor-readable recording medium, on which a computer program according to claim 11 is recorded.

13. A device (503) comprising a memory and a processor configured to implement the method for automated production of a variation curve of an indicator representing the heart rate variability of an individual or of a competition animal according to one of claims 1 to 9.

14. The device according to claim 13, further comprising:

    - means for receiving a request to establish said curve over a time period given by an individual;
    - means for transmitting said curve to a display screen.

101 ⟶ Détermination de plusieurs indices de forme sur une durée donnée

102 ⟶ Normalisation

103 ⟶ Interpolation 1

104 ⟶ Interpolation 2

105 ⟶ Filtrage

# FIG.1a

FIG.1b

FIG.2

EP 3 017 757 B1

301 ⌇ Acquisition mesures R-R

302 ⌇ Filtrage artefacts mesures

303 ⌇ Validation filtrage — non

oui

304 ⌇ Suppression effets de bords

305 ⌇ Construction sous-séquence 1    Construction sous-séquence 2 ⌇ 306

307 ⌇ Calcul indice

FIG.3

FIG.4a

EP 3 017 757 B1

FIG.4b

EP 3 017 757 B1

FIG.5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1407713 A1

**Littérature non-brevet citée dans la description**

- Heart rate variability. Standards of measurement, physiological interprétation and clinical use. American Heart Association Inc.: European Society of Cardiology, 1996
- **DAMIEN SABOUL et al.** The impact of breathing on HRV measurements: Implications for the longitudinal follow-up of athletes. *European Journal of Sport Science,* 2013
- **DANIEL J. PLEWS.** Training adaptation and heart rate variability in elite endurance athletes: opening the door to effective monitoring. Springer International Publishing Switzerland, 2013
- **FERDINANDO LELLAMO.** Conversion from vagal to sympathetic prédominance with strenuous training in high performance world class athletes. American Heart Association, 2002
- **GUZIK et al.** Correlations between the Poincaré Plot and conventional heart rate variability parameters assessed during paced breathing. *J.Physiol. Sci.,* Février 2007, vol. 57 (1), 63-71
- **AUBERT et al.** Heart rate variability in athletes. *Sports Med,* 2003